# EUROPEAN PATENT SPECIFICATION

(11) **EP 2 119 363 B1**
(45) Date of publication and mention of the grant of the patent: **09.01.2019**
(21) Application number: 09251265.6
(22) Date of filing: 06.05.2009
(51) Int. Cl.: A01N 65/12, A01N 65/22, A01N 65/24, A01N 65/28, A01N 65/34, A01N 33/00, A01N 49/00, A01N 59/26, A01P 1/00

(54) **Enhanced antimicrobial activity of plant essential oils**
Verstärkte antimikrobielle Wirkung von ätherischen Pflanzenölen
Amélioration de l'activité antimicrobienne d'huiles essentielles végétales

(30) Priority: 14.05.2008 US 53216 P
(43) Date of publication of application: 18.11.2009
(73) Proprietor: Van Beek, Ronald R., Orange City, IA 51041 (US)
(72) Inventor: Van Beek, Ronald R., Orange City, IA 51041 (US)
(74) Representative: Tomkinson, Alexandra

(56) References cited:
- WO-A1-96/00056
- WO-A1-99/66796
- WO-A2-96/37210
- WO-A2-2004/076680
- DE-A1- 10 005 886
- DE-U1-202004 015 396
- DE-U1-202007 003 020
- US-A- 5 837 222
- US-A1- 2005 013 883
- Antonia Nostro: "Plant extracts with antimicrobial activity", ISC Antibiotics Chemotherapy - Newsletter of the International Society of Chemotherapy, vol. 7, no. 3 December 2003 (2003-12), pages 1-16, XP002658258, ISSN: 1366-5146 Retrieved from the Internet: URL:http://www.ischemo.org/abstracts/vol07 _no3.pdf [retrieved on 2011-09-09]

## Description

### CROSS-REFERENCE TO RELATED APPLICATIONS

This application claims priority under 35 U.S.C. § 119(e) to provisional application Serial No. 61/053,216 filed May 14, 2008.

### FIELD OF THE INVENTION

This invention relates to use of plant essential oils as antimicrobial compositions, and to enhancing their effectiveness by addition of antimicrobial enhancers and by use of combinations of essential oils.

### BACKGROUND OF THE INVENTION

It is known in the art that plant essential oils, that is oils derived from plants by distillation, expression or extraction may have antimicrobial activity when exposed to bacterial cells. Consumer acceptance of these essential oils is high because they usually have the fragrance of the plant from which they were extracted. When used for animal use, the animals do not commonly shy away from them because again, they have the odor of plants from which they were derived, and such odors are not unfamiliar to many animals.

Essential oils mixed with carriers have a lot of potential veterinary and human uses. For example in the veterinary world they may be used as teat dips, or disinfecting topicals for skin ulcers, for shampoos, for topical gels and creams, for anti-fungals, and even to be taken internally for use in the GI tract.

There is a continuing need for increasing the cellular uptake of plant essential oils in order to enhance their anti-bacterial effect. Some researchers have theorized that plant essential oils soften the walls of the bacteria then permeate them thus causing the enhanced anti-bacterial effect, (*see,* Vaara, "Agents That Increase the Permeability of the Outer Membrane", Microbiological Reviews, September 1992, Vol. 56(3); and Johnson U.S. Patent No. 6,319,958 that teaches addition of at least one sesquiterpenoid to advance the antimicrobial effect of antimicrobial compounds.

DE202004015396 discloses a blend of the essential oil oregano with lanolin for use against MRSA.

Antonia Nostro: "Antiobiotics Chemotherapy", Newsletter of the International Society of Chemotherapy, Vol. 7, no. 3, December 2003 (2003-12), page 7, ISSN 1366-5146; discloses the antimicrobial activity of oregano oil against organisms such as E-coli.

US2005/013883 discloses the use of oregano oil and thyme oil for curing peach leaf curl in trees.

WO2004/076680 discloses the use of oregano oil in combination with other essential plant oils, such as cinnamon, thymus etc to provide an anti-microbial agent.

WO96/37210 discloses a pharmaceutical composition containing etheric oil obtained from origanum vulgaris and thymus vulgaris.

DE202007003020 discloses a disinfectant composition including oregano oil and oil from sage, coriander, geranium, cinnamon or lemongrass.

WO96/00056 discloses a method of preparing natual oil containing emulsion and microcapsules for repelling lice. The composition includes sodium tripolyphosphate.

US5837222 discloses a liquid dental cleaning solution containing sodium tripolyphosphate.

WO99/66796 discloses use of a sesquiterpenoid to enhance microbial compound uptake in the form of antibiotics.

DE10005886 disloses a disinfectant composition including vegetable oil and an essential oil, such as oregano oil or cinnamon oil.

In the ever increasing efforts to enhance antimicrobial effectiveness Applicant has now discovered that a combination of plant essential oils provides increased enhancement; and moreover the combination of oils may be used with other known enhancers to even further maximize effectiveness.

Accordingly it is an object of the present invention to provide plant essential oil-derived antimicrobial compositions, that use a combination of essential oils, and a combination of enhancers also used with the oils, to achieve a maximized antimicrobial effect.

It is also an object of this invention to prepare a variety of different antimicrobial compositions based on the above discovery that are useful for veterinary or human use.

The method or means of accomplishing at least the above objectives will become apparent from the detailed description of the invention which follows hereinafter.

### BRIEF SUMMARY OF THE INVENTION

An antimicrobial composition based on plant essential oils comprising:
at least two plant essential oils as a major component having anti-microbial effectiveness,
characterized in that a first essential oil is oregano oil and a second essential oil is cinnamon; and
a small but antimicrobial enhancing effective amount of an enhancer selected from the group consisting of polyethyleneimine, paramethoxyphenyl ethylmethylamine or a polyphosphate enhancer.

### DETAILED DESCRIPTION OF THE PREFERRED EMBODIMENT

This invention relates to antimicrobial compositions derived from plant essential oils, more importantly a combination of essential oils to enhance antimicrobial effectiveness. The essential oil component may contain from 40% to 95% by weight oils, but is preferably from 50% to 90% by weight of a combination of essential oils. The most preferred essential oils being a 1 to 1 by weight mix of the at least two different essential oils. As a major component as herein defined means at least 40% by weight.

Essential oils are volatile aromatic oils which may be synthetic or may be derived from plants by distillation, expression or extraction, and which usually carry the odor or
flavor of the plant from which they are obtained. In the combination compositions of this invention, antiseptic activity is provided by essential oils. Some of these essential oils also act as flavoring agents. Besides oregano oil and cinnamon oil, the essential oils of this invention may include but are not limited to menthol, methyl salicylate (wintergreen oil), eucalyptol, carvacrol, camphor, anethole, carvone, eugenol, isoeugenol, limonene, osimen, n-decyl alcohol, citronel, a-salpineol, methyl acetate, citronellyl acetate, methyl eugenol, cineol, linalool, ethyl linalaol, safrola vanillin, spearmint oil, peppermint oil, lemon oil, orange oil, sage oil, rosemary oil, , pimento oil, laurel oil, cedar leaf oil, thymol and clove oil.

In embodiments of the invention wherein organic phenolic compounds are obtained from plant oil extracts, the oil is preferably extracted from a member of the *Labiatae* (also called *Lamiaceae*) or *Verbenaceae* family. Plants in the family *Labiatae* or *Verbenaceae* include hybrids of plants produced from individual plants in those two families.

The common name for members of the *Labiatae* family, a large family of mostly annual or perennial herbs, is the "mint family." The mint family is classified in the division Magnoliphyta, class Magnoliopsida, and order Lamiales. The *Labiatae* family includes about 200 genera, such as *Salvia, Rosmarinus, Mentha, Ocimum, Thymus, Marrubium, Monarda, Trichostema, Teucrium, Hyptis, Physostegia, Lamium, Stachys, Scutellaria* and *Lycopus.*

Plants which are preferably used for extraction of organic phenolic compounds include, but are not limited to, *Ocimum* spp., *Saturea* spp., *Monarda* spp, *Origanum* spp, *Thymus* spp., *Mentha* spp., *Nepeta* spp., *Teucrium gnaphalodes, Teucrium polium, Teucrim divaricatum, Teucrim kotschyanum, Micromeria myrifolia, Calamintha nepeta, Rosmarinus officinalis, Myrtus communis, Acinos suaveolens, Dictamnus albus, Micromeria fruticosa, Cunila origanoides, Mosla Japonoica Maxymowitz, Pycnanthemum nudum, Micromeria Juliana, Piper betel, Trachyspermum ammi, Lippia graveolens Escholcia splendens,* and *Cedrelopsis grevei,* as well as others.

In a preferred composition, the oil is extracted from *Esholtia splendens, Cedrelopsis grevei, Lippia graveolens* or a plant of the species *Nepeta,* including but not limited to *Nepeta racemosa* (catmint), *Nepeta citriodora, Nepeta elliptica, Nepeta hindostoma, Nepeta lanceolata, Nepeta leucophylla, Nepeta longiobracteata, Nepeta mussinii, Nepeta nepetella, Nepeta sibthorpii, Nepeta subsessilis* and *Nepeta tuberosa.*

Most preferably, the oil is extracted from a hybrid plant produced from crossing *Nepeta racemosa, Esholtia splendens, Cedrelopsis grevei,* and *Lippia graveolens.*

Plants of the *Labiatae* and *Verbenacea* families are found throughout the world and are relatively easy to cultivate. To cultivate the plants, seeds, preferably those of plants that are expected to yield a high percentage (e.g., at least about 70 wt %, more preferably at least about 80 wt %), of organic phenolic compounds, are planted in fine loose soil, preferably in a sub-tropical climate. Hybrid seeds having a high percentage of organic phenolic compounds can be produced by known techniques. Crossing *Nepeta racemosa, Esholtia splendens, Cedrelopsis grevei,* and *Lippia graveolens* produces one such hybrid that is a preferred source of the organic phenolic compounds. The seeds are then cultivated using known agricultural techniques, such as watering, and artificial fertilizing. Most preferably, the plants are cultivated and grown without the use of any synthetic pesticides.

Because the leaves contain a high amount of oil upon blossoming, it is preferred that the plants be harvested soon after the plants begin to blossom. Preferably, the plants are harvested within 24 hours after blossoming, more preferably within 12 hours after blossoming. Most preferably, harvesting is undertaken early in the morning or late in the evening hours (after blossoming begins) when the leaves are not exposed to the sun.

Because the majority of the oil is found in the leaves and blossoms of the plant, it is preferred that only the leaves and blossoms be utilized in the extraction process. Use of other parts of the plant may increase impurities and decrease yield, but may be utilized.

Thymol, also known by the chemical formula 5-methyl-2-(1-methylethyl) phenol, is obtained from the essential oil of *Thymus vulgaris Labiatae* and *Monarda punctata Labiatae.* Thymol is a white crystalline powder with an aromatic odor and taste and is soluble in organic solvents but only slightly soluble in deionized water.

Menthol is isolated principally from the oil of Mentha arvensis. In its commercial form, menthol is available as L-menthol crystals obtained from a process involving cooling of the oil. Fractional distillation of peppermint oil which usually contains from about 40% to about 65% menthol represent another important source of menthol. Synthetic sources of L-menthol are also available.

Eucalyptol, another essential oil with antiseptic properties, is derived from the eucalyptus tree. Having a camphoraceous odor and cooling taste, this essential oil is often combined with other essential oils such as menthol in confection formulations to impart medicinal effect. Combinations of menthol and eucalyptol are widely used. Particularly preferred uses of the menthol-eucalyptol combination include, according to the present invention, dentifrices such as toothpastes or dental gels.

Methyl salicylate is the main ingredient in many essential oils, constituting about 99% of oil of wintergreen (*Gaultheria procumbens*) and sweet birch (*Betula lenta*). Methyl salicylate, which has a distinctive refreshing aroma, is used widely in mouthwashes, chewing gums and other oral and pharmaceutical preparations.

In the most preferred compositions of the present invention it is preferred that at least one of the essential oils have as their active ingredient a combination of thymol and carvacrol. One very satisfactory oil blend is 47.5% by weight oregano oil, 23.75% by weight cinnamon bark oil, and 23.75% by weight clove oil and 5% capsicum oil resin. Other oil blends may also be used such as: 46% by weight oregano, 22% cinnamon bark, 22% clove, 5% nerolidol and 5% capsicum.

A third blend formulation is 30% oregano, 30% cinnamon bark, 30% clove, 5% nerolidol and 5% capsicum.

A fourth blend formulation is 36.20% oregano, 18% cinnamon bark, 17% clove, 4% nerolidol, .8% oleoresin capsicum, 4% cranberry, 6.60% geranium, 6.67% patchouli, and 6.67% tea tree.

A fifth blend formulation is 33% regular oregano, 33.34% clove, and 33.34% cinnamon.

The essential oil can be mixed in a variety of physical formats, with one preferred one being so called beads. Bead format is from 0.5% to 50% of a combination of oils or pure oil added to a mixture of alginate, shellac and seaweed carriers to provide a carrier bead with the oil. This allows for a convenient and easy subsequent processing. The process is known and can be accomplished by a variety of manufacturers.

Products based on essential oils, such as those containing organic phenolic compounds, tend to be absorbed at a level greater than 90% in the small intestines. Therefore, most of the activity of such products tends to be localized in the stomach and/or small intestine. However, there are many microbial infections that occupy portions of the gastrointestinal tract beyond the small intestine. Therefore, it may be desirable to extend the activity of the combination essential oil based product into the large intestine.

Microencapsulation is one method that can help extend the-activity of the antimicrobial composition throughout the entire gastro-intestinal tract (GIT). Microencapsulation is a micro-packaging technique which involves the coating of small particles of solids, liquid droplets, or dispersion of solids, within liquids. Microencapsulated antimicrobial compound may be used to treat infections located in the end of the small intestines (e.g., jejunum and/or ileum) and beginning of the large intestines (e.g., ascending colon and transverse colon). The microencapsulation prevents release of the active ingredients in the stomach or in the beginning of the small intestines (e.g., duodenum). If the antimicrobial compound is not microencapsulated, the acidic environment of the stomach will tend to break the association between the antimicrobial compound and most carriers in the pharmaceutical composition (such as dextrose, starch, etc.) and thereby activate the antimicrobial compound in the stomach.

For example, a microencapsulated form of the antimicrobial compositions may be used to treat Cryptosporidia spp. infections and/or chronic enteritis in humans; Cryptosporidia infections in animals, *Lawsonia intracellularis* and *Treponema hyodesynteriae* infections in pigs, and others.

One example of a microencapsulation process includes encapsulating the antimicrobial composition in a multi walled capsule such that the layers of the wall dissolve as the capsule travels through the gastrointestinal tract. Thus, the components that make up each layer of the capsule wall are chosen based on the conditions in the specific region of the gastrointestinal tract in which they are desired to dissolve. For example, the pH along the gastrointestinal tract (GIT) varies: in the stomach, the pH is between 2 and 5; in the duodenum, 4 and 6; jejunum, 4 and 6; ileum, 6.5 and 7.5; caecum 5.5 and 6.5; colon, 6.5 and 7; and rectum, 6.5 and 7. Therefore, the components of the wall layers may differ depending on what type of an ailment is to be treated, or its location, and whether the final formulation is meant to treat humans or animals. Each layer of the wall may also contain the composition of the invention so that upon dissolution of that wall layer, it can be released to effectuate treatment of the ailment.

Suitable coating matrices include fatty acids, waxes, sugars, and shellac.

Encapsulation techniques are known. An example of one encapsulation technique (called fluidized bed coating) is provided below. In a fluidized bed, a suspension of solid particles is transformed into a fluid-like state by an upward gas flow through the system. Because of the intensive heat and mass transfer, fluidized bed reactors are widely used, e.g. in chemical industry for solid-catalyzed gas-phase reactions. To maximize the yield of such reactors, liquid reactants can locally be injected into the fluidized bed. The injected liquid reactants penetrate the fluidized bed and evaporate. For design purposes and the achievement of optimal operating conditions, the spatial distribution of the concentration of components and temperature has to be predicted.

Fluidized bed coating can be used to encapsulate the antimicrobial compound in a coating material which includes ethyl cellulose and plant oil. First, the antimicrobial compound described is combined in the fluid bed mixer with the ingredients to form a powder, such as the ingredients shown in the table below.

The especially preferred two-oil blend formulas include the following:

| 1^{st} Oil Blend Formula: | |
|---|---|
| Base Formula* | 33.34% |
| Regular Oregano Oil | 33.34% |
| Rosemary Oil | 11.11% |
| Licorice Powder | 11.11% |
| Cinnamon Bark | 11.11% |
| | 100% |

| 2^{nd} Oil Blend Formula: | |
|---|---|
| Base Formula* | 33.34% |
| Regular Oregano Oil | 33.34% |
| Cinnamon Bark Oil | 11.11% |
| Rosemary Oil | 11.11% |
| Peppermint Oil (high menthol content) | 11.11% |
| | 100% |

| | |
|---|---|
| * Base Formula as used here includes 33% Geranium oil, 33.34% Patchouli and 33.34 Tea Tree oil. | |

As used in the appended claims, major component refers to at least 40% by weight of the amount of combined essential oil component; minors include other additives.

The polymeric polyionic organic enhancer can be the preferred polyethyleneimine (PEI) or can be others such as paramethoxyphenyl ethylmethylamine. The amount can be 0.1 mM to 50mM, similar to the same amount of the sesquiterpenoids (herein before described).

The polyionic inorganic enhancers preferably polyphosphate enhancers and can include sodium tripolyphosphate, sodium hexametaphosphate, at similar levels.

Other carriers may include minors used for a variety of purposes in various topicals, pills, gelatins, etc. and can include small amounts of Apple Powder, Citrus Pectins, Arabic Gum, Ascorbic Acid, Beeswax, Betaine Hydrochloride, Biotin, Calcium Carbonate (Thermocal), Canola Oil, Cetyl Alcohol, Choline Chloride, Citric Acid, Cobalt Carbonate, Copper Sulfate, Corn Starch, Dextrose, Dry Sweet Orange Flavoring, Flaxseed Oil, Folic Acid, Glycine, Lanolin, Lavendar Oil, Lemon Powder, Lipase DS, Maltodextrin, Manganese Sulfate, Magnesium Chloride, Magnesium Oxide, Malic Acid, Niacin, Olive Oil, Pantothenic Acid, Potassium Chloride, Potassium Sulfate, Polysorbate, Propylene Glycol, Purple Pigment, Pyridoxine HCL, Riboflavin, Seaweed Meal, Probiotics/Bacteria, Selenium, Silicon 50S, Silicon Dioxide, Sodium Acetate, Sodium Chloride, Sodium Citrate, Sodium Propinate, Sodium Silica Aluminate - MS, Spearmint Oil, SST (Activated Charcaol), Steryl Alcohol, Thiamine, Vaseline, Vitamin A, Vitamin B12 600mg, Vitamin D3 500, Vitamin E, Vitamin K, Water, and Zinc Sulfate.

Applicants have also discovered that further anti-microbial property enhancement is achieved if from 0.01% by weight to 10% by weight, preferably 0.05% by weight to 5.0% by weight of an organic acid selected from the group of acetic, citric and fumaric is added to the oil combination containing composition.

Testing of the combination of oils at Iowa State University during the year 2006/2007 revealed that the combination of oils was more effective from an antimicrobial standpoint than the single oils alone. Preferred was the oil blend previously described as the first oil blend and the second oil blend. These oils in combination with various carriers may be used to make a variety of veterinarian and human use compositions, as previously stated and those include pills, gelatin capsules, skin topicals, gels, creams, liquid rub-ons, powders and shampoos and G.I. tract medicines..

It therefore can be seen that the invention accomplishes at least all of its stated objectives.

## Claims

1. An antimicrobial composition based on plant essential oils, comprising:
at least two plant essential oils as a major component having anti-microbial effectiveness,
**characterised in that** a first essential oil is oregano oil and a second essential oil is cinnamon oil; and
an antimicrobial enhancing effective amount of an enhancer selected from the group
consisting of polyethyleneimine, paramethoxyphenyl ethylmethylamine or a polyphosphate enhancer.

2. The anti-microbial composition of claim 1 wherein the enhancer is a polyionic polyphosphate enhancer selected from the group consisting of sodium tripolyphosphate and sodium hexametaphosphate.

3. The anti-microbial composition of claim 1 which also includes at least one sesquinterpenoid.

4. The antimicrobial composition of claim 3 wherein the at least one sesquiterpenoid is selected from the group consisting of farnesol, nerolidol, bisabolol and apritone.

5. The anti-microbial composition of claim 3 wherein the amount of sesquiterpenoid is from 0.1mM to 50mM.

6. The anti-microbial composition of claim 1 wherein said composition is microencapsulated.

7. The anti-microbial composition of claim 6 wherein said composition is encapsulated in a multi walled capsule.

8. The anti-microbial composition of claim 1 wherein the composition contains from 0.01%
by weight to 10% by weight of an organic acid selected from the group consisting of acetic acid, citric acid and fumaric acid.

9. The anti-microbial composition of claim 1 wherein the composition contains from 0.05% by weight to 5% by weight of an organic acid selected from the group consisting of acetic acid, citric acid and fumaric acid.

10. An antimicrobial composition for use as an effective anti-microbial in the treatment of microbial infections in the gastrointestinal tract of humans or animals comprising:
a combination of at least two plant essential oils having anti-microbial effectiveness, **characterised in that** a first essential oil is oregano oil and a second essential oil is cinnamon oil, and a cellular uptake enhancing effective amount of an enhancer selected from the group consisting of polyethyleneimine, paramethoxyphenyl ethylmethylamine or a polyphosphate enhancer.

11. The anti-microbial composition for use according to claim 10 wherein the combination of essential oils and enhancer are microencapsulated in a multi-walled capsule.

12. The anti-microbial composition for use according to claim 10 wherein the components of the multi-walled capsule are chosen based upon the specific region of the gastrointestinal tract in which they are desired to dissolve.

13. The anti-microbial composition for use according to claim 10 wherein the composition is used to treat microbial infections of Cryptosporidia spp and/or chronic enteritis in humans, Cryptosporidia infections in animals, *Lawsonia intracellularis* infections in pigs or *Treponema hyodesynteriae* infections in pigs.

## Patentansprüche

1. Antimikrobielle Zusammensetzung auf der Basis von pflanzlichen ätherischen Ölen, die Folgendes umfasst:
wenigstens zwei pflanzliche ätherische Öle als Hauptkomponente mit antimikrobieller Wirksamkeit, **dadurch gekennzeichnet, dass** ein erstes ätherisches Öl Oreganoöl und ein zweites ätherisches Öl Zimtöl ist; und
eine wirksame Menge eines Enhancers zur Verstärkung eines antimikrobiellen Mittels, der ausgewählt ist aus der Gruppe bestehend aus Polyethylenimin, Paramethoxyphenylethylmethylamin oder einem Polyphosphat-Enhancer.

2. Antimikrobielle Zusammensetzung nach Anspruch 1, wobei der Enhancer ein polyionischer Polyphosphat-Enhancer ist, ausgewählt aus der Gruppe bestehend aus Natriumtripolyphosphat und Natriumhexametaphosphat.

3. Antimikrobielle Zusammensetzung nach Anspruch 1, die wenigstens auch ein Sesquiterpenoid enthält.

4. Antimikrobielle Zusammensetzung nach Anspruch 3, wobei das wenigstens eine Sesquiterpenoid ausgewählt ist aus der Gruppe bestehend aus Farnesol, Nerolidol, Bisabolol und Apriton.

5. Antimikrobielle Zusammensetzung nach Anspruch 3, wobei die Menge an Sesquiterpenoid 0,1 mM bis 50 mM beträgt.

6. Antimikrobielle Zusammensetzung nach Anspruch 1, wobei die genannte Zusammensetzung mikroeingekapselt ist.

7. Antimikrobielle Zusammensetzung nach Anspruch 6, wobei die genannte Zusammensetzung in einer mehrwandigen Kapsel eingekapselt ist.

8. Antimikrobielle Zusammensetzung nach Anspruch 1, wobei die Zusammensetzung 0,01 Gew.-% bis 10 Gew.-% einer organischen Säure enthält, die aus der Gruppe bestehend aus Essigsäure, Zitronensäure und Fumarsäure ausgewählt ist.

9. Antimikrobielle Zusammensetzung nach Anspruch 1, wobei die Zusammensetzung 0,05 Gew.-% bis 5 Gew.-% einer organischen Säure enthält, die aus der Gruppe bestehend aus Essigsäure, Zitronensäure und Fumarsäure ausgewählt ist.

10. Antimikrobielle Zusammensetzung zur Verwendung als wirksames antimikrobielles Mittel bei der Behandlung von mikrobiellen Infektionen im Magen-Darm-Trakt von Menschen oder Tieren, die Folgendes umfasst:
eine Kombination von wenigstens zwei pflanzlichen ätherischen Ölen mit antimikrobieller Wirksamkeit,
**dadurch gekennzeichnet, dass** ein erstes ätherisches Öl Oreganoöl und ein zweites ätherisches Öl Zimtöl ist, und eine wirksame Menge eines Enhancers zur Verstärkung der Zellaufnahme, der aus der Gruppe bestehend aus Polyethylenimin, Paramethoxyphenylethylmethylamin und einem Polyphosphat-Enhancer ausgewählt ist.

11. Antimikrobielle Zusammensetzung zur Verwendung nach Anspruch 10, wobei die Kombination von ätherischen Ölen und Enhancern in einer mehrwandigen Kapsel mikroeingekapselt ist.

12. Antimikrobielle Zusammensetzung zur Verwendung nach Anspruch 10, wobei die Komponenten der mehrwandigen Kapsel auf der Basis der spezifischen Region des Magen-Darm-Traktes, in der sie sich auflösen sollen, ausgewählt werden.

13. Antimikrobielle Zusammensetzung zur Verwendung nach Anspruch 10, wobei die Zusammensetzung zur Behandlung mikrobieller Infektionen von Cryptosporidia spp und/oder chronischer Enteritis in Menschen, Cryptosporidia-Infektionen in Tieren, *Lawsonia intracellularis*-Infektionen in Schweinen oder *Treponema hyodesynteriae-*Infektionen in Schweinen verwendet wird.

## Revendications

1. Composition antimicrobienne basée sur des huiles essentielles végétales, comprenant :
au moins deux huiles essentielles végétales en tant que composant majeur ayant une efficacité antimicrobienne **caractérisée en ce qu'**une première huile essentielle est une huile d'origan et une deuxième huile essentielle est une huile de cannelle ; et
une quantité efficace d'amélioration de l'activité antimicrobienne d'un additif alimentaire sélectionné dans le groupe constitué d'un additif alimentaire de polyéthylèneimine, d'éthylméthylamine de paraméthoxyphényle ou de polyphosphate

2. Composition antimicrobienne selon la revendication 1 dans laquelle l'additif alimentaire est un additif alimentaire de polyphosphate polyionique sélectionné dans le groupe constitué de tripolyphosphate de sodium et d'hexamétaphosphate de sodium.

3. Composition antimicrobienne selon la revendication 1 qui contient également au moins un sesquiterpénoïde.

4. Composition antimicrobienne selon la revendication 3 dans laquelle au moins un sesquiterpénoïde est sélectionné dans le groupe constitué de farnesol, de nérolidol, de bisabolol et d'apritone.

5. Composition antimicrobienne selon la revendication 3 dans laquelle la quantité de sesquiterpénoïde se situe dans l'intervalle allant de 0,1 mM à 50 mM.

6. Composition antimicrobienne selon la revendication 1, ladite composition étant microencapsulée.

7. Composition antimicrobienne selon la revendication 6, ladite composition étant encapsulée dans une capsule à plusieurs parois.

8. Composition antimicrobienne selon la revendication 1, la composition contenant de 0,01 % en poids à 10 % en poids d'un acide organique sélectionné dans le groupe constitué d'acide acétique, d'acide nitrique et d'acide fumarique.

9. Composition antimicrobienne selon la revendication 1, la composition contenant de 0,05 % en poids à 5 % en poids d'un acide organique sélectionné dans le groupe constitué d'acide acétique, d'acide nitrique et d'acide fumarique.

10. Composition antimicrobienne pour une utilisation comme antimicrobien efficace dans le traitement d'infections microbiennes dans le tube digestif d'êtres humains ou d'animaux comprenant :
une combinaison d'au moins deux huiles essentielles végétales ayant une efficacité antimicrobienne,
**caractérisée en ce qu'**une première huile essentielle est de l'huile d'origan et une deuxième huile essentielle est de l'huile de cannelle, et une quantité efficace d'amélioration de l'absorption cellulaire d'un additif alimentaire est sélectionnée dans le groupe constitué d'un additif de polyéthylèneimine, d'éthylméthylamine de paraméthoxyphényle ou d'un polyphosphate.

11. Composition antimicrobienne pour une utilisation selon la revendication 10 dans laquelle la combinaison d'huiles essentielles et d'additif alimentaire est microencapsulée dans une capsule à plusieurs parois.

12. Composition antimicrobienne pour une utilisation selon la revendication 10 dans laquelle les composants de la capsule à plusieurs parois sont choisis sur la base de la région spécifique du tube digestif dans lequel il est souhaitable qu'ils se dissolvent.

13. Composition antimicrobienne pour une utilisation selon la revendication 10, la composition étant utilisée pour traiter des infections microbiennes induites par Crystosporidia spp et/ou une entérite chronique chez des êtres humains, des infections à Crystosporidia chez des animaux, des infections à *Lawsonia intracellularis* chez des cochons ou des infections à *Treponema hyodesynteriae* chez des cochons.
